(19)

Europäisches
Patentamt
European
Patent Office
Office européen
des brevets

(11) **EP 4 620 391 B1**

(12) **EUROPEAN PATENT SPECIFICATION**

(45) Date of publication and mention
of the grant of the patent:
**15.04.2026   Bulletin 2026/16**

(21) Application number: **25164147.8**

(22) Date of filing: **17.03.2025**

(51) International Patent Classification (IPC):
*A61B 5/11* (2006.01)        *G16H 20/30* (2018.01)
*G16H 50/20* (2018.01)       *G16H 50/30* (2018.01)
*G16H 50/50* (2018.01)

(52) Cooperative Patent Classification (CPC):
**G16H 50/50; A61B 5/112; A61B 5/1121;
A61B 5/1124; A61B 5/1128; G16H 20/30;
G16H 50/20; G16H 50/30**

(54) **METHOD AND SYSTEM FOR PERSONALIZED AND OPTIMAL SELECTION OF ANKLE FOOT
ORTHOSIS**

VERFAHREN UND SYSTEM ZUR PERSONALISIERTEN UND OPTIMALEN AUSWAHL EINER
FUSSGELENKORTHESE

PROCÉDÉ ET SYSTÈME DE SÉLECTION PERSONNALISÉE ET OPTIMALE D'ORTHÈSE
CHEVILLE-PIED

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **20.03.2024   IN 202421021181**

(43) Date of publication of application:
**24.09.2025   Bulletin 2025/39**

(73) Proprietor: **Tata Consultancy Services Limited
Maharashtra (IN)**

(72) Inventors:
• **MAZUMDER, Oishee
700156 Kolkata (IN)**
• **SINHA, Aniruddha
700091 Kolkata (IN)**
• **PERWEEN, Tarannum
700091 Kolkata (IN)**

(74) Representative: **Goddar, Heinz J.
Boehmert & Boehmert
Anwaltspartnerschaft mbB
Pettenkoferstrasse 22
80336 München (DE)**

(56) References cited:
• **STEELE KATHERINE M ET AL: "How much
muscle strength is required to walk in a crouch
gait?", JOURNAL OF BIOMECHANICS, vol. 45,
no. 15, 27 July 2012 (2012-07-27), pages 2564 -
2569, XP028942523, ISSN: 0021-9290, DOI:
10.1016/J.JBIOMECH.2012.07.028**
• **ZHANG HAOTIAN ET AL: "A Framework of a
Lower Limb Musculoskeletal Model With
Implemented Natural Proprioceptive Feedback
and Its Progressive Evaluation", IEEE
TRANSACTIONS ON NEURAL SYSTEMS AND
REHABILITATION ENGINEERING, IEEE, USA,
vol. 28, no. 8, 19 June 2020 (2020-06-19), pages
1866 - 1875, XP011802331, ISSN: 1534-4320,
[retrieved on 20200806], DOI: 10.1109/
TNSRE.2020.3003497**
• **YAMAMOTO MASATAKA ET AL: "Effect of Ankle-
Foot Orthosis Stiffness on Muscle Force During
Gait Through Mechanical Testing and Gait
Simulation", IEEE ACCESS, IEEE, USA, vol. 9, 8
July 2021 (2021-07-08), pages 98039 - 98047,
XP011866466, DOI: 10.1109/
ACCESS.2021.3095530**

**Description**

CROSS-REFERENCE TO RELATED APPLICATIONS AND PRIORITY

The present application claims priority to Indian application no. 202421021181, filed on March 20, 2024.

TECHNICAL FIELD

The disclosure herein generally relates to an Ankle Foot Orthosis (AFO), and, more particularly, to a processor-implemented method, system, and machine-readable medium for processing and evaluating crouch-gait characteristics in individuals with Cerebral Palsy (CP).

BACKGROUND

Crouch gait is a pathological movement involving abnormal kinematics and muscle activation leading to inefficient and high energetic cost of walking, and commonly seen in children suffering from Cerebral Palsy (CP). The crouch gait is characterized by excessive flexion of hip, knee, and ankle during a stance phase of a gait. The cause and manifestation of the crouch gait are often multifactorial, arising because of muscle spasticity, impaired selective motor control, and contracture leading to weakness in ankle planter flexor and knee extensor muscles. The crouch gait usually progresses with age and growth, making treatment and disease management challenging. Treatment for the crouch gait involves selective muscle strengthening, physical therapy, spasticity reduction, use of an Ankle Foot Orthoses (AFOs), and surgical intervention to enable an efficient walking pattern.

Out of conventional treatment approaches to improve the crouch gait, use of passive AFOs remains one of most common interventions. The passive AFOs are prescribed to patients having weak plantar flexor or dorsiflexor muscles due to disorders like stroke, CP, spinal cord injury, and thereof. In CP children, passive AFOs are known to assist ankle dynamics and improve gait kinematics that prevent bone deformity and reduce the energy cost of walking. The passive AFOs provides ankle stabilization by allowing heel contact with the ground during the stance phase to maintain a stable posture and improves gait by preventing foot drop in the stance phase. The passive AFOs that resist ankle dorsiflexion are the most prescribed orthoses for children with the CP. Generation of a torque in the passive AFO is dependent on ankle kinematics and properties of the passive AFOs like stiffness and equilibrium angle. Judicious designing of the passive AFOs with optimal stiffness can potentially reduce the energetic cost of walking during the gait by modulating passive AFOs storage and release of mechanical energy in a gait cycle.

There are multiple variants of the passive AFOs comprising a solid AFO, a dynamic AFO, a hinged AFO, and thereof. The solid AFO counteract excessive knee flexion during the stance phase of gait and are known to normalize knee kinematics and kinetics effectively. However, the solid AFO is generally ineffective in reducing push-off power. A spring-like AFO, like leaf spring may enhance the push-off power but with limited reduction in knee flexion. Optimizing the trade-off between push-off power and flexion angle maximizes efficiency of the gait. Further the response of the subject in terms of the crouch gait improvement depends on selecting a correct AFO as well as optimal AFO setting. However, this is challenging as it is difficult to predict how a specific design of the AFO will impact muscle action and reduce the energy cost of walking for individual subjects.

Documents XP028942523 (STEELE KATHERINE M ET AL: "How much muscle strength is required to walk in a crouch gait?",JOURNAL OF BIOMECHANICS, vol. 45, no. 15 , pages 2564-2569),

XP011802331 (ZHANG HAOTIAN ET AL: "A Framework of a Lower Limb Musculoskeletal Model With Implemented Natural Proprioceptive Feedback and Its Progressive Evaluation",IEEE TRANSACTIONS ON NEURAL SYSTEMS AND REHABILITATION ENGINEERING, IEEE, USA, vol. 28, no. 8, 19 June 2020 (2020-06-19), pages 1866-1875) and

XP011866466 (YAMAMOTO MASATAKA ET AL: "Effect of Ankle-Foot Orthosis Stiffness on Muscle Force During Gait Through Mechanical Testing and Gait Simulation",IEEE ACCESS, IEEE, USA, vol. 9, 8 July 2021 (2021-07-08), pages 98039-98047) discloses a multi-stage, computational method for selecting an optimal Ankle-Foot Orthosis (AFO) controller for a Cerebral Palsy crouch-gait subject according to prior art.

SUMMARY

Embodiments of the present disclosure present technological improvements as solutions to one or more of the above-mentioned technical problems recognized by the inventors in conventional systems. For example, in one embodiment, a method for personalized and optimal selection of AFO is provided. The method includes receiving a motion-captured crouch gait data, a height, a body weight, and a severity of a crouch gait pertaining to a Cerebral Palsy (CP) subject. The method further includes computing a joint ankle angle kinematics comprising a plurality of joint ankle angles, from the motion-captured crouch gait data, at a plurality of three-dimensional (3D) ankle joint locations of the CP subject using an inverse kinematics pipeline. The method further includes feeding the plurality joint ankle angles, the height, and the body weight of the CP subject, to a plurality of Ankle Foot Orthosis (AFO) controllers, wherein each of the plurality of AFO controllers are programmed with associated mechanical behavior. The method further includes computing a corresponding AFO torque, by each of the plurality of AFO controllers in accordance with associated mechanical behavior. The method further includes integrating the generated AFO torque, and the plurality joint ankle angles of each of the plurality of AFO controllers with a musculoskeletal human lower limb model (MHLLM) comprising a human skeleton and a plurality of lower limb muscles, to generate an associated AFO integrated MHLLM, corresponding to each of the plurality of AFO controllers. The method further includes performing an inverse dynamics mechanism on the associated AFO integrated MHLLM, to compute an additional joint torque at an ankle joint of the human skeleton, corresponding to each of the plurality of AFO controllers. The method further includes computing a plurality of muscle forces corresponding to the plurality of lower limb muscles of the associated AFO integrated MHLLM, for a gait cycle of the CP subject, using a static optimization framework, corresponding to each of the plurality of AFO controllers. The method further includes computing a plurality of muscle response metrics comprising a muscle impulse, a muscle yank, a muscle co-activation, and an energetic cost of walking, from the plurality of muscle forces and the additional joint torque, corresponding to each of the plurality of AFO controllers. The method further includes combining the plurality of muscle response metrics, to generate an AFO selector score, corresponding to each of the plurality of AFO controllers. The method further includes ranking the plurality of AFO controllers in increasing order based on the AFO selector scores. The method further includes selecting a top ranked AFO controller as a personalized optimal AFO controller from among the plurality of AFO controllers for the CP subject.

In another aspect, a system for personalized and optimal selection of AFO is provided. The system comprising: a memory storing instructions; one or more communication interfaces; and one or more hardware processors coupled to the memory via the one or more communication interfaces, wherein the one or more hardware processors are configured by the instructions to: receive a motion-captured crouch gait data, a height, a body weight, and a severity of a crouch gait pertaining to a Cerebral Palsy (CP) subject; computing a joint ankle angle kinematics comprising a plurality of joint ankle angles, from the motion-captured crouch gait data, at a plurality of three-dimensional (3D) ankle joint locations of the CP subject using an inverse kinematics pipeline; feed the plurality joint ankle angles, the height, and the body weight of the CP subject, to a plurality of Ankle Foot Orthosis (AFO) controllers, wherein each of the plurality of AFO controllers are programmed with associated mechanical behavior; compute a corresponding AFO torque, by each of the plurality of AFO controllers in accordance with associated mechanical behavior; integrate the generated AFO torque, and the plurality joint ankle angles of each of the plurality of AFO controllers with a musculoskeletal human lower limb model (MHLLM) comprising a human skeleton and a plurality of lower limb muscles, to generate an associated AFO integrated MHLLM, corresponding to each of the plurality of AFO controllers; perform an inverse dynamics mechanism on the associated AFO integrated MHLLM, to compute an additional joint torque at an ankle joint of the human skeleton, corresponding to each of the plurality of AFO controllers; compute a plurality of muscle forces corresponding to the plurality of lower limb muscles of the associated AFO integrated MHLLM, for a gait cycle of the CP subject, using a static optimization framework, corresponding to each of the plurality of AFO controllers; compute a plurality of muscle response metrics comprising a muscle impulse, a muscle yank, a muscle co-activation, and an energetic cost of walking, from the plurality of muscle forces and the additional joint torque, corresponding to each of the plurality of AFO controllers; combine the plurality of muscle response metrics, to generate an AFO selector score, corresponding to each of the plurality of AFO controllers; rank the plurality of AFO controllers in increasing order based on the AFO selector scores; and select a top ranked AFO controller as a personalized optimal AFO controller from among the plurality of AFO controllers for the CP subject based on the ranking.

In yet another aspect, there are provided one or more non-transitory machine-readable information storage mediums comprising one or more instructions, which when executed by one or more hardware processors causes a method for personalized and optimal selection of AFO is provided. The method includes receiving a motion-captured crouch gait data, a height, a body weight, and a severity of a crouch gait pertaining to a Cerebral Palsy (CP) subject. The method further includes computing a joint ankle angle kinematics comprising a plurality of joint ankle angles, from the motion-captured crouch gait data, at a plurality of three-dimensional (3D) ankle joint locations of the CP subject using an inverse kinematics pipeline. The method further includes feeding the plurality joint ankle angles, the height, and the body weight of the CP subject, to a plurality of Ankle Foot Orthosis (AFO) controllers, wherein each of the plurality of AFO controllers are programmed with associated mechanical behavior. The method further includes computing a corresponding AFO torque, by each of the plurality of AFO controllers in accordance with associated mechanical behavior. The method further includes integrating the generated AFO torque, and the plurality joint ankle angles of each of the plurality of AFO controllers with a musculoskeletal human lower limb model (MHLLM) comprising a human skeleton and a plurality of lower limb muscles, to generate an associated AFO integrated MHLLM, corresponding to each of the plurality of AFO controllers. The method further includes performing an inverse dynamics mechanism on the associated AFO integrated MHLLM, to compute an additional joint torque at an ankle joint of the human skeleton, corresponding to each of the plurality of AFO controllers. The method further includes computing a plurality of muscle forces corresponding to the plurality of lower limb muscles of the associated AFO integrated MHLLM, for a gait cycle of the CP subject, using a static optimization framework, corresponding to each of the plurality of AFO controllers. The method further includes computing a plurality of muscle response metrics comprising a muscle impulse, a muscle yank, a muscle co-activation, and an energetic cost of walking, from the plurality of muscle forces and the additional joint torque, corresponding to each of the plurality of AFO controllers. The method further includes combining the plurality of muscle response metrics, to generate an AFO selector score, corresponding to each of the plurality of AFO controllers. The method further includes ranking the plurality of AFO controllers in increasing order based on the AFO selector scores. The method further includes selecting a top ranked AFO controller as a personalized optimal AFO controller from among the plurality of AFO controllers for the CP subject.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for a personalized and optimal selection of Ankle Foot Orthosis (AFO), in accordance with some embodiments of the present disclosure.
FIG. 2 is a functional architecture depicting process flow of the system for the personalized and the optimal selection of the AFO, in accordance with some embodiments of the present disclosure.
FIGS. 3A, and 3B depict a flow diagram of a method for the personalized and the optimal selection of the AFO, using the system of FIG. 1, in accordance with some embodiments of the present disclosure.
FIGS. 4A, 4B, and 4C depict a plurality of muscle forces across a severity of a crouch gait for an assisted walking and an unassisted walking, in accordance with some embodiments of the present disclosure.
FIG. 5 depicts a muscle impulse calculated for a plurality of muscles across the severity of the crouch gait, in accordance with some embodiments of the present disclosure.
FIG. 6 depicts a muscle yank calculated for the plurality of muscles across the severity of the crouch gait, in accordance with some embodiments of the present disclosure.
FIG. 7 depicts a muscle co-activation calculated for the plurality of muscles across the severity of crouch gait, in accordance with some embodiments of the present disclosure.
FIG. 8 depicts an energy cost of walking calculated for the plurality of muscles across the severity of crouch gait, in accordance with some embodiments of the present disclosure.

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

An Ankle Foot Orthosis (AFO) is commonly prescribed for correcting a crouch gait in children with cerebral palsy (CP). There are multiple AFO variants, however selecting an optimal AFO for a CP subject is often challenging.

Embodiments herein provide a method and system for personalized and optimal selection of the AFO. The AFO is also referred to as an AFO controller, in accordance with some embodiments of the present disclosure. The disclosed method focusses on selecting the personalized and the optimal selection of an AFO controller using an AFO torque, and a plurality joint ankle angles of each of a plurality of AFO controllers integrated with a musculoskeletal human lower limb model (MHLLM). A plurality of muscle forces is computed using the MHLLM for each of the plurality of AFO controllers. Further the method computes a plurality of muscle response metrics comprising a muscle impulse, a muscle yank, a muscle co-activation, and an energetic cost of walking, from the plurality of muscle forces and an additional joint torque for each of the AFO controllers. The plurality of muscle response metrics is combined to obtain a personalized optimal AFO

**EP 4 620 391 B1**

controller among the plurality of AFO controllers of the CP subject.

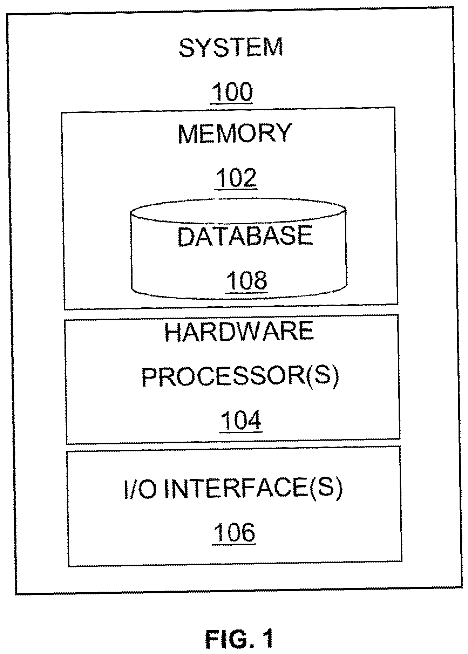

Referring now to the drawings, and more particularly to FIG. 1 through FIG. 8, where similar reference characters denote corresponding features consistently throughout the figures, there are shown preferred embodiments, and these embodiments are described in the context of the following exemplary system and/or method.

FIG. 1 is a functional block diagram of a system 100 for the personalized and the optimal selection of the AFO, in accordance with some embodiments of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors.

Referring to the components of the system 100, in an embodiment, the processor (s) 104 can be the one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the processor(s) 104 is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface (s) 106 can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. Thus, the memory 102 may comprise information pertaining to input(s)/output(s) of each step performed by the processor(s) 104 of the system 100 and methods of the present disclosure. In an embodiment, a database 108 is comprised in the memory 102, wherein the database 108 comprises information on a motion-captured crouch gait data, a height, a body weight, and a severity of the crouch gait. The memory 102 further comprises a plurality of modules (not shown) for various technique(s) such as inverse kinematics pipeline, the MHLLM, a static optimization framework and thereof. The above-mentioned technique(s) are implemented as at least one of a logically self-contained part of a software program, a self-contained hardware component, and/or, a self-contained hardware component with a logically self-contained part of a software program embedded into each of the hardware component (e.g., hardware processor 104 or memory 102) that when executed perform the method described herein.

The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

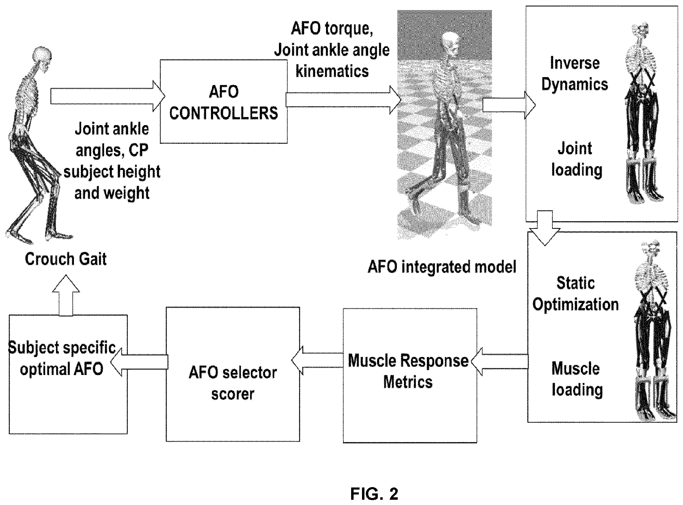

FIG. 2 is a functional architecture depicting process flow of the system 100 for the personalized and the optimal selection of the AFO, in accordance with some embodiments of the present disclosure. The system 100 in FIG. 2 is configured to receive the motion-captured crouch gait data, the height, and the body weight pertaining to the CP subject and is fed to the plurality of AFO controllers. The plurality of AFO controllers refers to various AFO devices available in a market. The AFO controllers block is configured to compute a joint ankle angle kinematics comprising a plurality of joint ankle angles, and the AFO torque for each of the AFO controllers. The AFO torque is a force exerted by rotational movements. The plurality of joint ankle angles and the AFO torque of each of the plurality of AFO controllers are integrated with the MHLLM to generate an integrated MHLLM for each of the plurality of AFO controllers. A joint loading block is configured to perform inverse dynamics on the associated AFO integrated MHLLM to compute the additional joint torque at the plurality of joint angles corresponding to each of the plurality of AFO controllers. A muscle loading block is configured to compute the plurality of muscle forces corresponding to a plurality of lower limb muscles of the AFO integrated MHLLM, for a gait cycle of the CP subject, using the static optimization framework, corresponding to each of the plurality of AFO controllers. One gait cycle is defined as a heel strike to next the heel strike of a same leg. A muscle response block is configured to the plurality of muscle response metrics comprising the muscle impulse, the muscle yank, and the muscle co-activation, and the energetic cost of walking, from the plurality of muscle forces and the additional joint torque, corresponding to each of the plurality of AFO controllers. An AFO selector scorer block is configured to combine the plurality of muscle response metrics, to generate an AFO selector score, corresponding to each of the plurality of AFO controllers. A subject specific AFO block is configured to select the personalized optimal AFO controller from among the plurality of AFO controllers for the CP subject based on the ranking.

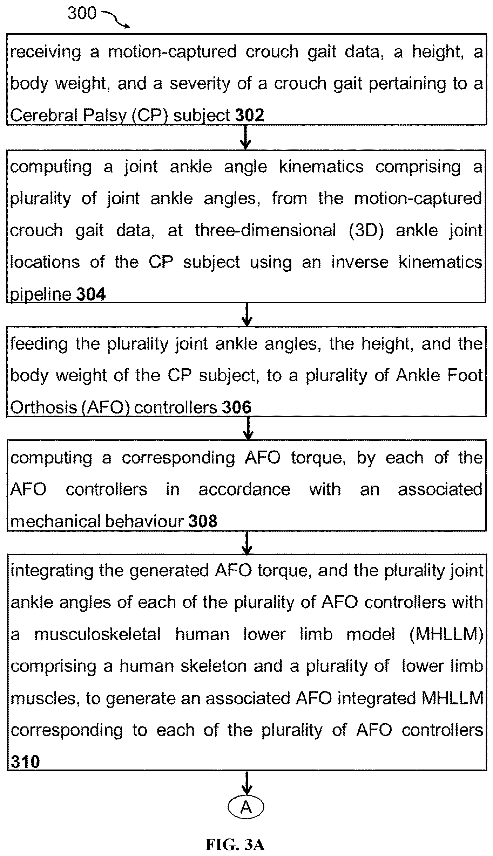

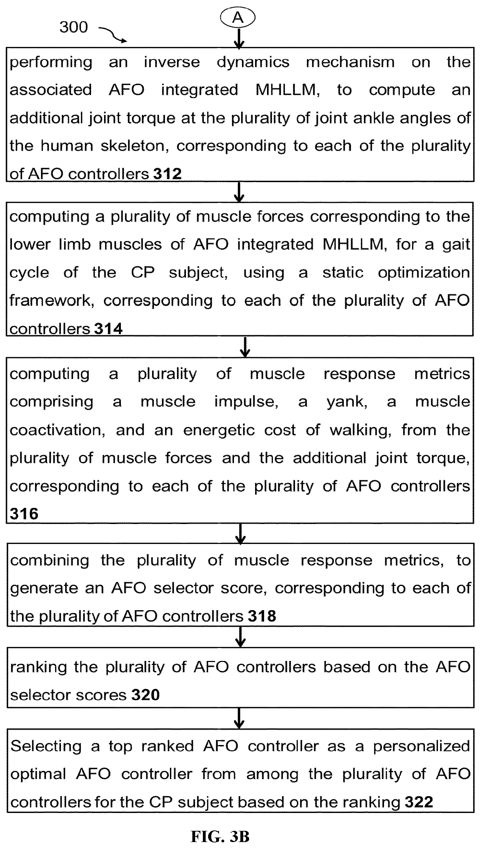

FIGS. 3A, and 3B depict a flow diagram of a method 300 for the personalized and the optimal selection of the AFO,

5

using the system of FIG. 1, in accordance with some embodiments of the present disclosure.

In an embodiment, the system 100 comprises one or more data storage devices or the memory 102 operatively coupled to the processor(s) 104 and is configured to store instructions for execution of steps of the method 300 by the processor(s) 104. The steps of the method 300 of the present disclosure will now be explained with reference to the components or blocks of the system 100 as depicted in FIG. 1, the functional architecture depicted in FIG. 2, and the steps of flow diagram as depicted in FIGS. 3A, and 3B. Although process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

Referring to steps of FIG. 3A, at step 302 of the method 300, the one or more hardware processors are configured to receive the motion-captured crouch gait data, the height, the body weight, and the severity of the crouch gait pertaining to the CP subject. The motion-captured crouch gait data is used from a repository, generated from motion analysis data. The motion-captured crouch gait comprises kinematics and ground reaction force data for the CP subject. The severity of the crouch gait is based on knee flexion angle (KFA) during a stance phase of the gait. The severity of the crouch gait comprises one of (i) a normal gait, (ii) a mild crouch gait, (iii) a moderate crouch gait, and (iv) a severe crouch gait.

At step 304 of the method 300, the one or more hardware processors are configured to compute the joint ankle angle kinematics comprising the plurality of joint ankle angles, from the motion-captured crouch gait data, at a plurality of three-dimensional (3D) ankle joint locations of the CP subject using inverse kinematics pipeline. The inverse kinematics pipeline takes the plurality of 3D ankle joint locations and returns the joint ankle angle kinematics comprising the plurality of joint ankle angles. This is computed by a weighted least square optimization that minimizes error of optical markers used to record the plurality of 3D ankle joint locations. An inverse dynamics tool of the inverse kinematics pipeline goes through each time step frame of motion and computes generalized coordinate values which positions the MHLLM in a pose that best matches experimental marker and the coordinate values for that time step. Mathematically, the best match is expressed as a weighted least squares problem, whose solution aims to minimize both the marker and errors of the coordinate values.

At step 306 of the method 300, the one or more hardware processors are configured to feed the plurality joint ankle angles, the height, and the body weight of the CP subject, to the plurality of AFO controllers, wherein each of the plurality of AFO controllers are programmed with associated mechanical behavior. The mechanical behavior of the AFO controllers refers to a torque generation principle of the AFO controller and is defined by combination of an optimal stiffness value and an AFO equilibrium angle. Therefore, the mechanical behavior of each of the plurality of AFO controllers is composed as combination of the optimal stiffness value, and the AFO equilibrium angle, for generating the corresponding AFO torque.

At step 308 of the method 300, the one or more hardware processors are configured to compute the corresponding AFO torque, by each of the plurality of AFO controllers in accordance with the associated mechanical behavior. The AFO torque generated by each of the plurality of AFO controllers are modelled and guide the joint ankle kinematics of the CP subject along with the optimal stiffness value (K) and the AFO equilibrium angle ($\theta_{eq}$). The AFO equilibrium angle is an angle between a foot plate of the AFO controller and a shank plate at which the AFO controller starts generating the AFO torque. The plurality of AFO controllers comprises a Ground Reaction AFO (GRAFO), and Leaf Spring AFO (LSAFO), in accordance with some embodiments of the present disclosure. The AFO torque generated by the GRAFO is given by:

$$\tau_{GRAFO} = \begin{cases} k(\theta_{ank} - \theta_{eq}) & if\ \theta_{ank} > \theta_{eq} \\ 0 & other\ wise \end{cases} \qquad (1)$$

where k is the optimal stiffness value, $\theta_{eq}$ is the AFO equilibrium angle, and $\theta_{ank}$ is the plurality of joint ankle angles. The AFO torque is generated during dorsiflexion only. In a planter flexion, the j the plurality of joint ankle angles is kept fixed. The optimal value of stiffness is used as k = 3.66N/m, and the equilibrium angle $\theta_{eq}$ = 6deg).

The AFO torque generated by the LSAFO has two components, a planter flexion (PF) torque, and a dorsiflexion (DF) torque.

$$\tau_{LSAFO} = \begin{cases} k_s((UL - \theta_{eq}) - \theta_{ank}) + \frac{k_d}{w_d} & if\ PF \\ k_s((LL + \theta_{eq}) - \theta_{ank}) + \frac{k_d}{w_d} & if\ DF \end{cases} \qquad (2)$$

The term $\frac{k_d}{w_d}$ represents a damping AFO torque, $k_d = 0.2N/m$ is a damping coefficient, $w_d$ is a joint velocity. Upper Limit (UL) is the joint ankle angle of the plurality of joint ankle angles above which the LSAFO generates the PF torque, Lower Limit (LL) is the joint ankle angle of the plurality of joint ankle angles above which the LSAFO generates the DF torque. The UL and the LL are tunable parameters that are adjusted from the CP subject specific joint ankle angle of the plurality of joint ankle angles during the gait. The optimal stiffness value and the equilibrium angle used are: $k_s = 2.7N/m$, $\theta_{eq} = 4deg$.

At step 310 of the method 300, the one or more hardware processors are configured to integrate the generated AFO torque, and the plurality joint ankle angles of each of the plurality of AFO controllers with the MHLLM comprising a human skeleton and the plurality of lower limb muscles, to generate the associated AFO integrated MHLLM, corresponding to each of the plurality of AFO controllers. The MHLLM is designed based on the severity of the crouch gait.

At step 312 of the method 300, the one or more hardware processors are configured to perform an inverse dynamics mechanism on the associated AFO integrated MHLLM, to compute the additional joint torque at the plurality of joint ankle angles of the human skeleton, corresponding to each of the plurality of AFO controllers. The additional joint torque is computed based on the AFO equilibrium angle, the optimal stiffness value, and the plurality of joint ankle angles of the CP subject.

The additional joint torque for each of the plurality of AFO controllers are computed as:

$$\tau = M(q)\ddot{q} + C(q, \dot{q}) + G(q) + F \qquad (3)$$

*where, $\ddot{q}$, $\dot{q}$, and q, represents an acceleration, a velocity, and a position due to the AFO torque $\tau$,*
*M is a mass matrix,*
*C and G are a Coriolis component and gravity component, and*
*F is any external force applied to the* MHLLM.

At step 314 of the method 300, the one or more hardware processors are configured to compute the plurality of muscle forces corresponding to the plurality of lower limb muscles of the associated AFO integrated MHLLM, for the gait cycle of the subject CP subject, using the static optimization framework, corresponding to each of the plurality of AFO controllers. The static optimization framework uses the known motion of the MHLLM to solve the equations of motion for the unknown generalized force of joint torques at respective joints like hip, knee, joint ankle angles, and thereof.

The plurality of muscle forces generated in the AFO integrated MHLLM are based on a Thelen muscle actuator, where the plurality of muscle forces or a AFO torque component($\tau_m$) is calculated as $\tau_m = [R(q)] f(a, l, i)$. $[R(q)]$ is a moment arm, $a$ is an activation value, and $l$ is a normalized length of muscle unit. The plurality of muscle forces over one gait cycle is estimated using the static optimization framework. The plurality of muscle forces is estimated by minimizing sum of squared muscle activations, generated using a Thelen muscle model that is required to drive experimentally captured kinematics and ground reaction force at each time instance.

At step 316 of the method 300, the one or more hardware processors are configured to compute the plurality of muscle response metrics comprising the muscle impulse, the muscle yank, the muscle co-activation, and the energetic cost of walking, from the plurality of muscle forces and the additional joint torque, corresponding to each of the plurality of AFO controllers. A plurality of muscles considered for computing the plurality of muscle response metrics comprises Gluteus muscle (GM) (combination of medius, maximus and minimus), Illiopsas (ILL), Rectus Femoris (RF), Vastus group (VAS), Hamstring group (HAM) comprising bicep femoris, semimembranosus and semitendinosus muscles, Soleus (SOL), Tibialis anterior (TA) and Gastrocnemius (GAS).

The muscle impulse is computed as a sum of the plurality muscle forces for each of the plurality of muscles integrated over the stance phase of the gait cycle, and is expressed as:

$$muscle\ impulse = \frac{1}{BW}\sum F\ dt \qquad (4)$$

Where BW is the body weight, F is the muscle force, and t is a duration of the stance phase of the gait cycle. This is equivalent to a mechanical work rate of the plurality of muscles and is a major component of metabolic cost.

The muscle yank, also referred to as Rate of force development (RFD) is an important biomechanics metric that correlates with different responses linked with sensori-motor system. The muscle yank is a derivative of force with respect to time that can be used in measurement of time variation of propulsive force during movements ranging from locomotion to responses of sensory organs that are used in motor reflexes. Athletic performance and recovery during rehabilitation are shown to be closely related to improvement in the RFD. The RFD or the muscle yank parameter evaluation for the plurality of muscles provides intuitive information about efficiency in selection of the AFO. The muscle yank is expressed as:

$yank = \frac{\partial f}{\partial t}$ , where F is the plurality of muscle forces, t is the duration of the stance phase of the gait cycle.

The muscle co-activation is a ratio of agonist-antagonist muscle pair, which is an important metric in movement mechanics and dictates joint stability, stiffness, rate of movement, and thereof. The muscle co-activation is evaluated around the plurality of joint ankle angles, considering Soleus and Tibialis anterior muscles as the agonist-antagonist muscle pair to compare changes in the muscle co-activation.

A key metric in for evaluating the performance of the plurality of AFO controllers is the energetic cost of walking. Metabolic cost calculation is conventionally measured using indirect calorimetry, by measuring the volume of oxygen-inspired and carbon dioxide expired. The MHLLM can be alternately used to estimate an energy expenditure based on a metabolic energy expenditure model which uses several surrogate markers like muscle and joint power and energy, computed from changes in muscle length and the muscle co-activation, in accordance with some embodiments of the present disclosure. The energetic cost of walking is expressed as:

$$energetic\ cost\ of\ walking = \frac{1}{Tmv}\int_0^T \sum_{i=1}^N \dot{E}_i\ dt \qquad (5)$$

Where $T$ is the duration of the stance phase of the gait cycle, $mv$ is a mass and speed of the CP subject, N is the plurality of muscles, $\dot{E}_i$ is energy rate of muscle $i$.

At step 318 of the method 300, the one or more hardware processors are configured to combine the plurality of muscle response metrics, to generate the AFO selector score, corresponding to each of the plurality of AFO controllers. The plurality of response metric comprising the muscle impulse, the muscle yank, the muscle co-activation, and the energetic cost of walking combined to obtain the score that could guide towards selection of the personalized optimal AFO controller. A cost function for the selection of the personalized optimal AFO controller is defined as:

$$J = E_n * y_n' * I_n' * C_n \qquad (6)$$

Where $E_n$ is a normalized energetic cost of walking, $y_n'$ is a normalized ratio of the muscle yank computed between the soleus and the TA muscle, $I_n'$ is a normalized ratio of the muscle impulse computed between the soleus and the TA muscle, $C_n$ is a normalized muscle co-activation.

At step 320 of the method 300, the one or more hardware processors are configured to rank the plurality of AFO controllers based on the AFO selector scores in increasing order of the cost function.

At step 322 of the method 300, the one or more hardware processors are configured to select a top ranked AFO controller as the personalized optimal AFO controller from among the plurality of AFO controllers for the CP subject based on the ranking. The selection of the personalized optimal AFO controller based on the ranking that returns minimum value of J.

**Experimental Details**

The motion-captured crouch gait data is used from a repository, generated from motion analysis data captured at Gillette Childrens Specialty Healthcare. It consists of kinematics and ground reaction force data of three typically developing (TD) children and nine children with diplegic CP and crouch gait with varying severity. Severity grading was based on knee flexion angle (KFA) during the stance phase of the gait. The subjects were asked to walk barefoot overground at self-selected speed. Out of total of 12 subject data, 1 TD subject and 3 CP subjects, representing the mild crouch gait, the moderate crouch gait, a severe crouch gait. Subject metadata are provided in Table.I

Table. I

| Subject Parameters | | | | | |
|---|---|---|---|---|---|
| **Severity** | **Age (years)** | **Weight(kg)** | **Height(m)** | **Speed(m/sec)** | **KFA (degree)** |
| TD | 10.2 | 41.1 | 1.45 | 1.01 | 1.4 |
| Mild | 9.4 | 28.2 | 1.31 | 0.94 | 15.5 |
| Moderate | 8.7 | 21.1 | 1.31 | 0.9 | 33.1 |
| Severe | 13.2 | 35.9 | 1.44 | 0.8 | 60.4 |

The MHLLM is used for analysis of the crouch gait that was adapted from a pre-existing full-body model in Opensim consisting of 92 muscle actuators and 19 degrees of freedom in torso and lower extremities. Arm motion was not considered for analysis of the crouch gait. The MHLLM is scaled as per anthropomorphic data of each child under assessment.

Inverse kinematics (IK) pipeline was used to convert the motion-captured crouch data to the plurality of joint ankle angles. Residual forces were adjusted using a residual reduction algorithm for missing upper body arm motion and the torso mass centre was re-adjusted based on body segment accelerations and measured ground reaction forces. The additional Joint torques are calculated using the inverse dynamics mechanism, using joint trajectory generated from the inverse kinematics pipeline and the measured ground reaction force. The plurality of muscle forces generated in the MHLLM are based on a Thelen muscle actuator.

Ground or floor reaction AFOs are plastic moulded custom-fabricated solid AFO (SAFO), providing ankle support and stability by reducing ankle dorsiflexion and excessive knee flexion in the stance phase of gait. The GRAFO is generally effective in controlling tibial advancement over the foot in midstance phase, improving knee extension utilizing the plantarflexion or knee extension couple concept. The GRAFO can be readily used for CP, flat foot correction, posterior tibial tendon dysfunction, osteoarthritis, spinal cord injuries, and thereof. Posterior leaf spring orthosis (LSAFO) is a modified SAFO, with a characteristic trimline located behind the ankle and a leaf-shaped corrugation or creases near the ankle. The creases act like spring, allowing slight dorsiflexion in the mid and terminal stance. Energy returned from spring assist in push-off in terminal stance. The LSAFO are generally used for motor weakness in ankle dorsiflexors due to CP or stroke. The foot plate and the shank plate support of the GRAFO and the LSAFO were designed in Solidworks utilizing standard dimensions and weights. The design was imported in the Opensim and scaled for specific subjects under analysis. The AFO foot plate was fixed to the calcaneous and the shank plate was fixed to tibia of the MHLLM model. The AFO Torque generated by the plurality of AFO were modelled, guided by the subject-specific joint ankle angle kinematics along with the optimal stiffness value and the equilibrium angle, which is the angle between the AFO footplate and the shank plate at which the AFO starts generating the AFO torque. once the AFO is fixed to the joint ankle angle of the plurality of joint ankle angles, an AFO angle is considered same as the sagittal plane ankle motion throughout the gait cycle, derived from IK pipeline in the MHLLM model. The AFO torque generated by the plurality of AFOs were integrated in Opensim model dynamics.

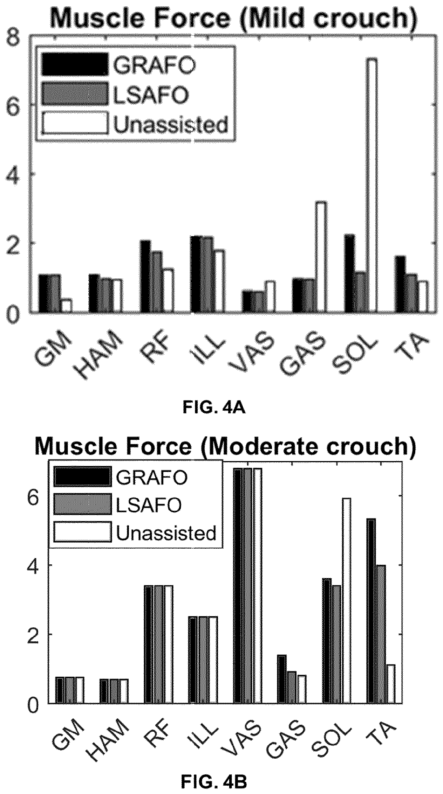

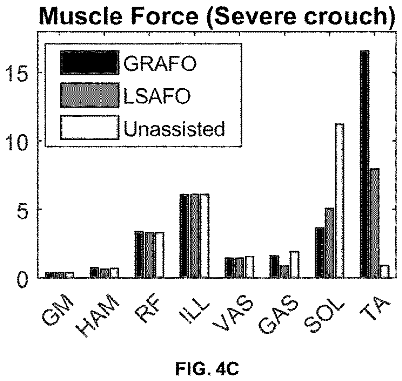

Unassisted walking for the TD subject, and assisted walking with the GRAFO and the LSAFO as well as unassisted walking for subjects with crouch gait were simulated and analyzed for one gait cycle. The plurality of muscle forces and the plurality muscle response metrics are computed only for the stance phase of the gait cycle. FIGS. 4A, 4B, and 4C depict a plurality of muscle force across the severity of the crouch gait the assisted walking and the unassisted walking, in accordance with some embodiments of the present disclosure. More specifically Fig.4 depicts a mean muscle force variation through crouch severity, scaled with respect to the body weight for the assisted walking with the GRAFO, the LSAFO, and the unassisted walking. Table. II shows the plurality of muscle response metrics muscle parameters for the TD child as a reference.

Table.II

| Muscle response metrics for TD subject | | | | | | | |
|---|---|---|---|---|---|---|---|
| **Muscles** | GM | HAM | RF | ILL | VAS | GAS | SOL | TA |
| **Muscle Force (N)** | 0.97 | 0.38 | 0.5 | 2.5 | 0.88 | 0.56 | 3.5 | 1.2 |
| **Muscle Impulse (Nsec)** | 0.66 | 0.25 | 0.2 | 1.9 | 0.25 | 0.3 | 2.8 | 0.3 |
| **Muscle Yank (N/sec)** | 0.03 | 0.13 | 0.4 | 0.4 | 1.68 | 2.7 | 0.57 | 2.1 |

Compared to the normal gait (TD), subjects walking in the crouch gait utilized higher muscle forces, and recruitment force increased as severity increased. As knee flexion increases during the crouch gait, there is a greater demand for the plurality of muscle forces to support and propel the body. This is most prominent in quadriceps group (combination of RF and VAS muscle group). The difference between the assisted walking and the unassisted walking was mostly reflected in the planter flexor (SOL, GAS) and dorsiflexor muscles (TA), indicating that the use of the AFO, both solid and spring type does not affect knee or hip muscles directly. The AFO torque generated by the GRAFO and the LSAFO mostly translates in controlling Soleus (PF) and TA (DF) muscle pair, by increasing DF force and decreasing PF force. The GRAFO produced high TA force compared to LSAFO. GRAFO torque operates only during DF phase, hence its effect on DF muscle is more prominent than the PF counterpart. Soleus muscle control is better in LSAFO, providing reduction in muscle force across crouch severity. However, muscle behavior across severity for the GRAFO and the LSAFO are not consistent, following different trends for the mild crouch gait, the moderate crouch gait, and the severe crouch gait.

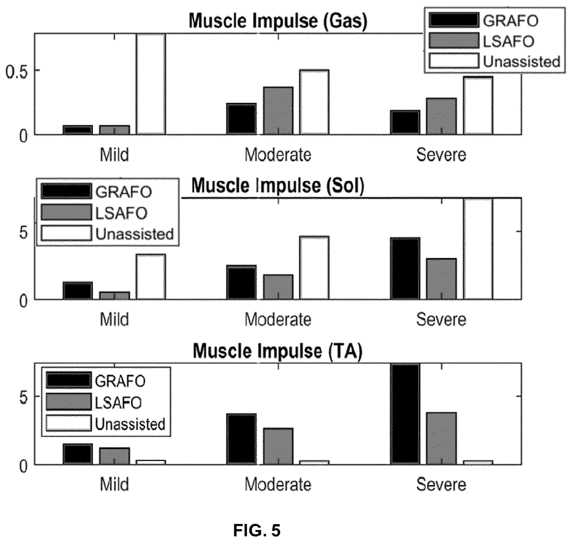

It is noticed that changes in plurality of muscle forces are prominent in PF-DF muscle pair. FIG. 5 depicts the muscle impulse for the plurality of muscles across the severity of crouch gait, in accordance with some embodiments of the present disclosure. More specifically FIG. 5 depicts the muscle impulse for the GAS, the SOL, and the TA across the severity of crouch gait, in accordance with some embodiments of the present disclosure.

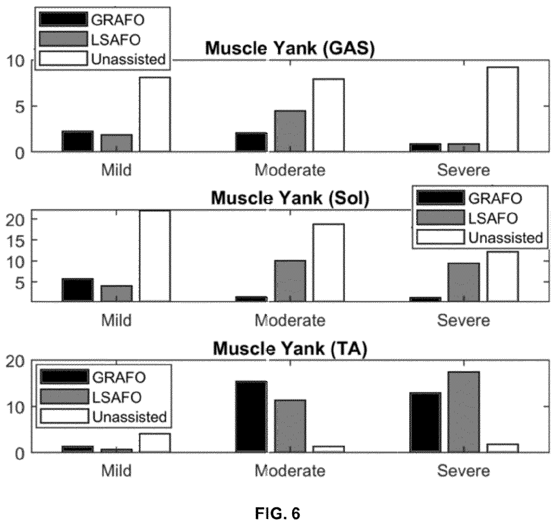

FIG. 6 depicts muscle yank for a plurality of muscles across the severity of crouch gait, in accordance with some embodiments of the present disclosure. More specifically FIG. 6 depicts the muscle yank for the GAS, the SOL, and the TA across the severity of crouch gait, in accordance with some embodiments of the present disclosure.

The values of the muscle impulse and the muscle yank during the stance phase, are scaled with respect to the BW for the TD subject for all the plurality of muscles provided in Table II. The muscle impulse follows an increasing trend across the severity of the crouch gait. Compared to the unassisted walking, both types of assisted walking reduced PF muscle impulse and increased DF (TA) impulse. For mild gait crouch, GAS impulse was almost similar for the GRAFO and the LSAFO, but for the moderate crouch gait and the severe crouch gait, the GRAFO decreased GAS impulse to a greater extent. But for Soleus muscle, in all the three severity groups, the LSAFO reduced impulse to a greater extent compared to the GRAFO. For TA, the assistance by the GRAFO provided increased muscle impulse compared to the LSAFO.

The muscle yank shows a decreasing trend for the PF muscles in assisted walking compared to the unassisted and an increasing trend for the DF muscles. An increase in magnitude of the muscle yank refers to a high rate of force development, associated with activities where takeoff velocity needs to be maximized like jumping or leaping. Higher value of the muscle yank supports higher speed of walking or running and the high muscle yank value in the DF muscles with the AFO could better stabilize the ankle with increase in speed. In between severity groups, the GRAFO reported lower rate of force development for the SOL muscle and the GAS muscle. For the TA muscle, the GRAFO produced a higher muscle yank value for the moderate crouch gait while the LSAFO performed better in the severe crouch gait.

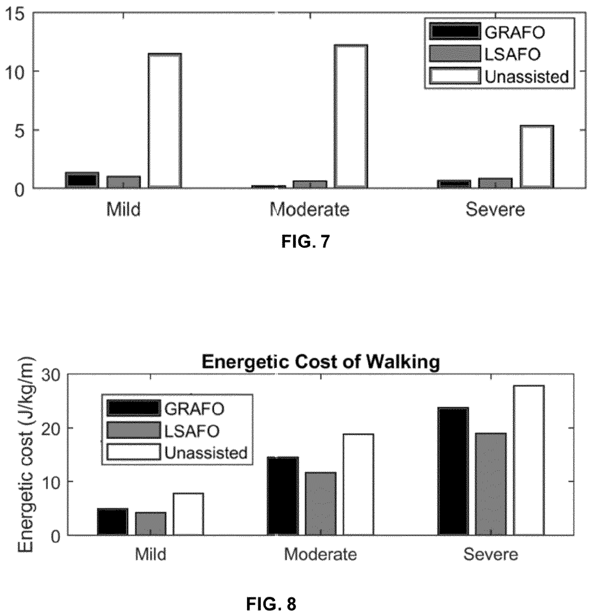

FIG. 7 depicts co-activation calculated for the plurality of muscles across the severity of crouch gait, in accordance with some embodiments of the present disclosure. More specifically FIG. 7 depicts the muscle co-activation calculated for ankle joint movement considering the SOL and the TA muscle pair for GRAFO, LSAFO and unassisted walking across varying severity. The muscle co-activation is reduced with the use of the GRAFO, the LSAFO compared to unassisted walking. Lower muscle co-activation refers to lower joint stability but an improved rate of motion due to decreased stiffness. FIG. 8 depicts an energy cost of walking calculated for the plurality of muscles across the severity of crouch gait, in accordance with some embodiments of the present disclosure. The crouch gait is a result of biomechanical alteration to decrease the energetic cost of walking. As reduction in the energetic cost of walking is of primal importance compared to joint stability, a decrease in the muscle co-activation is desired. For the mild crouch gait and severe crouch gait, the LSAFO performance was better compared to the GRAFO. The energetic cost of walking increases with the severity of the crouch gait. Between the GRAFO and the LSAFO, the energetic cost of walking is least in the LSAFO across the crouch severity due to the additional spring-induced push-off power generation.

Analysis of the plurality of various muscle response metrics for the plurality of AFO controllers of AF the GRAFO and the LSAFO across the severity of the crouch gait indicates the effectiveness of using AFO in modifying crouch dynamics. In terms of energetic cost of walking, the LSAFO showed maximum efficacy, while for the other muscle response metrics, the LSAFO and the GRAFO performance varied with change in severity of the crouch gait. Results suggest that the personalized optimal AFO controller, dictated by the crouch gait and muscle dynamics of the CP subject could provide the best performance. Understanding of muscle behavior could also aid in prescribing muscle-strengthening activities that could help CP subject to walk in a more upright posture.

The experimental results indicate improvement in the energetic cost of walking using the LSAFO across the severity of the crouch gait, whereas indices like the muscle impulse, the muscle yank, and the muscle co-activation were better with the GRAFO in some cases and LSAFO in other cases. The analysis of muscle response metrics could aid in optimizing selection of the AFO that results in lower muscle demand and metabolic cost based on subject-specific gait dynamics. The disclosed method apart from selecting the personalized optimal AFO can also aid in identifying potential muscles for training and strengthening that could reduce muscle spasticity, stiffness and weakness associated with the CP or other musculoskeletal disorders.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

Embodiments herein provide a method and system for personalized and optimal selection of AFO. There are multiple AFO variants, however selecting the optimal AFO for a CP subject is often challenging. The disclosed method focusses on selecting personalized and optimal selection of the AFO controller using the AFO torque, and the plurality joint ankle angles of each of the plurality of AFO controllers integrated with the MHLLM. The plurality of muscle forces is computed using the MHLLM for each of the plurality of AFO controllers. Further the method computes the plurality of muscle response metrics comprising the muscle impulse, the muscle yank, the muscle co-activation, and the energetic cost of walking, from the plurality of muscle forces and the additional joint torque for each of the AFO controllers. The plurality of muscle response metrics is combined which enables the selection of the personalized optimal AFO controller among the plurality of AFO controllers of the CP subject.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300), the method comprising:

   receiving (302), via a one or more hardware processors, a motion-captured crouch gait data, a height, a body weight, and a severity of a crouch gait pertaining to a Cerebral Palsy (CP) subject;
   computing (304), via the one or more hardware processors, a joint ankle angle kinematics comprising a plurality of joint ankle angles, from the motion-captured crouch gait data, at a plurality of three-dimensional (3D) ankle joint locations of the CP subject using an inverse kinematics pipeline;
   feeding (306), via the one or more hardware processors, the plurality joint ankle angles, the height, and the body weight of the CP subject, to a plurality of Ankle Foot Orthosis (AFO) controllers, wherein each of the plurality of AFO controllers are programmed with associated mechanical behavior;
   computing (308), by the one or more hardware processors, a corresponding AFO torque, by each of the plurality of AFO controllers in accordance with the associated mechanical behavior;
   integrating (310), by the one or more hardware processors, the generated AFO torque, and the plurality joint ankle angles of each of the plurality of AFO controllers with a musculoskeletal human lower limb model (MHLLM) comprising a human skeleton and a plurality of lower limb muscles, to generate an associated AFO integrated MHLLM, corresponding to each of the plurality of AFO controllers;

performing (312), by the one or more hardware processors, an inverse dynamics mechanism on the associated AFO integrated MHLLM, to compute an additional joint torque at the plurality of ankle joint angles of the human skeleton, corresponding to each of the plurality of AFO controllers;

computing (314), by the one or more hardware processors, a plurality of muscle forces corresponding to the plurality of lower limb muscles of the associated AFO integrated MHLLM, for a gait cycle of the CP subject, using a static optimization framework, corresponding to each of the plurality of AFO controllers;

computing (316), by the one or more hardware processors, a plurality of muscle response metrics comprising a muscle impulse, a muscle yank, a muscle co-activation, and an energetic cost of walking, from the plurality of muscle forces and the additional joint torque, corresponding to each of the plurality of AFO controllers;

combining (318), by the one or more hardware processors, the plurality of muscle response metrics, to generate an AFO selector score, corresponding to each of the plurality of AFO controllers;

ranking (320), by the one or more hardware processors, the plurality of AFO controllers in increasing order based on the AFO selector scores; and

selecting (322), by the one or more hardware processors, a top ranked AFO controller as a personalized optimal AFO controller from among the plurality of AFO controllers for the CP subject.

2. The processor implemented method as claimed in claim 1, wherein the mechanical behavior of each of the plurality of AFO controllers is composed as combination of an optimal stiffness value, and an AFO equilibrium angle, for generating the corresponding AFO torque.

3. The processor implemented method as claimed in claim 1, wherein the additional joint torque is computed based on the AFO equilibrium angle, the optimal stiffness value, and the plurality of joint ankle angles of the CP subject.

4. The processor implemented method as claimed in claim 1, wherein the MHLLM is designed based on the severity of the crouch gait.

5. The processor implemented method as claimed in claim 1, wherein the severity of the crouch gait comprises one of (i) a normal gait, (ii) a mild crouch gait, (iii) a moderate crouch gait, and (iv) a severe crouch gait.

6. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive a motion-captured crouch gait data, a height, a body weight, and a severity of a crouch gait pertaining to a Cerebral Palsy (CP) subject;

compute a joint ankle angle kinematics comprising a plurality of joint ankle angles, from the motion-captured crouch gait data, at a plurality of three-dimensional (3D) ankle joint locations of the CP subject using an inverse kinematics pipeline;

feed the plurality joint ankle angles, the height, and the body weight of the CP subject, to a plurality of Ankle Foot Orthosis (AFO) controllers, wherein each of the plurality of AFO controllers are programmed with associated mechanical behaviour;

compute a corresponding AFO torque, by each of the plurality of AFO controllers in accordance with the associated mechanical behaviour;

integrate the generated AFO torque, and the plurality joint ankle angles of each of the plurality of AFO controllers with a musculoskeletal human lower limb model (MHLLM) comprising a human skeleton and a plurality of lower limb muscles, to generate an associated AFO integrated MHLLM, corresponding to each of the plurality of AFO controllers;

perform an inverse dynamics mechanism on the associated AFO integrated MHLLM, to compute an additional joint torque at an ankle joint of the human skeleton, corresponding to each of the plurality of AFO controllers;

compute a plurality of muscle forces corresponding to the plurality of lower limb muscles of the associated AFO integrated MHLLM, for a gait cycle of the CP subject, using a static optimization framework, corresponding to each of the plurality of AFO controllers;

compute a plurality of muscle response metrics comprising a muscle impulse, a muscle yank, a muscle co-activation, and an energetic cost of walking, from the plurality of muscle forces and the additional joint torque, corresponding to each of the plurality of AFO controllers;

combine the plurality of muscle response metrics, to generate an AFO selector score, corresponding to each of the plurality of AFO controllers;

rank the plurality of AFO controllers in increasing order based on the AFO selector scores; and

select a top ranked AFO controller as a personalized optimal AFO controller from among the plurality of AFO controllers for the CP subject based on the ranking.

7. The system as claimed in claim 6, wherein the mechanical behavior of each of the plurality of AFO controllers is composed as combination of an optimal stiffness value, and an AFO equilibrium angle, for generating the corresponding AFO torque.

8. The system as claimed in claim 6, wherein the additional joint torque is computed based on the AFO equilibrium angle, the optimal stiffness value, and the plurality of joint ankle angles of the CP subject.

9. The system as claimed in claim 6, wherein the MHLLM is designed based on the severity of the crouch gait.

10. The system as claimed in claim 6, wherein the severity of the crouch gait comprises one of (i) a normal gait, (ii) a mild crouch gait, (iii) a moderate crouch gait, and (iv) a severe crouch gait.

11. One or more non-transitory machine-readable information storage mediums comprising one or more instructions which when executed by one or more hardware processors cause:

receiving a motion-captured crouch gait data, a height, a body weight, and a severity of a crouch gait pertaining to a Cerebral Palsy (CP) subject;

computing a joint ankle angle kinematics comprising a plurality of joint ankle angles, from the motion-captured crouch gait data, at a plurality of three-dimensional ankle joint locations of the CP subject using an inverse kinematics pipeline;

feeding the plurality joint ankle angles, the height, and the body weight of the CP subject, to a plurality of Ankle Foot Orthosis (AFO) controllers, wherein each of the plurality of AFO controllers are programmed with associated mechanical behavior;

computing a corresponding AFO torque, by each of the plurality of AFO controllers in accordance with the associated mechanical behavior;

integrating the generated AFO torque, and the plurality joint ankle angles of each of the plurality of AFO controllers with a musculoskeletal human lower limb model (MHLLM) comprising a human skeleton and a plurality of lower limb muscles, to generate an associated AFO integrated MHLLM, corresponding to each of the plurality of AFO controllers;

performing an inverse dynamics mechanism on the associated AFO integrated MHLLM, to compute an additional joint torque at the plurality of ankle joint angles of the human skeleton, corresponding to each of the plurality of AFO controllers;

computing a plurality of muscle forces corresponding to the plurality of lower limb muscles of the associated AFO integrated MHLLM, for a gait cycle of the CP subject, using a static optimization framework, corresponding to each of the plurality of AFO controllers;

computing a plurality of muscle response metrics comprising a muscle impulse, a muscle yank, a muscle co-activation, and an energetic cost of walking, from the plurality of muscle forces and the additional joint torque, corresponding to each of the plurality of AFO controllers;

combining the plurality of muscle response metrics, to generate an AFO selector score, corresponding to each of the plurality of AFO controllers;

ranking the plurality of AFO controllers in increasing order based on the AFO selector scores; and

selecting a top ranked AFO controller as a personalized optimal AFO controller from among the plurality of AFO controllers for the CP subject.

12. The one or more non-transitory machine-readable information storage mediums as claimed in claim 11, wherein the mechanical behavior of each of the plurality of AFO controllers is composed as combination of an optimal stiffness value, and an AFO equilibrium angle, for generating the corresponding AFO torque.

13. The one or more non-transitory machine-readable information storage mediums as claimed in claim 11, wherein the additional joint torque is computed based on the AFO equilibrium angle, the optimal stiffness value, and the plurality of joint ankle angles of the CP subject.

14. The one or more non-transitory machine-readable information storage mediums as claimed in claim 11, wherein the MHLLM is designed based on the severity of the crouch gait.

15. The one or more non-transitory machine-readable information storage mediums as claimed in claim 11, wherein the severity of the crouch gait comprises one of (i) a normal gait, (ii) a mild crouch gait, (iii) a moderate crouch gait, and (iv) a severe crouch gait.

**Patentansprüche**

1. Prozessorimplementiertes Verfahren (300), wobei das Verfahren Folgendes umfasst:

Empfangen (302), über einen oder mehrere Hardwareprozessoren, von bewegungserfassten Schrittgangdaten, einer Höhe, eines Körpergewichts und einer Schwere eines Schrittgangs, der sich auf eine Person mit zerebraler Lähmung (CP) bezieht;

Berechnen (304), über den einen oder die mehreren Hardwareprozessoren, einer Gelenkknöchelwinkelkinematik, die eine Mehrzahl von Gelenkknöchelwinkeln umfasst, aus den bewegungserfassten Schrittgangdaten an einer Mehrzahl von dreidimensionalen (3D) Knöchelgelenkpositionen der CP-Person unter Verwendung einer inversen Kinematik-Pipeline;

Zuführen (306), über den einen oder die mehreren Hardwareprozessoren, der Mehrzahl von Gelenkknöchelwinkeln, der Höhe und des Körpergewichts der CP-Person zu einer Mehrzahl von Steuerungen der Knöchelfußorthese (AFO), wobei jede der Mehrzahl von AFO-Steuerungen mit einem zugehörigen mechanischen Verhalten programmiert ist;

Berechnen (308), durch den einen oder die mehreren Hardwareprozessoren, eines entsprechenden AFO-Drehmoments durch jede der Mehrzahl von AFO-Steuerungen gemäß dem zugehörigen mechanischen Verhalten;

Integrieren (310), durch den einen oder die mehreren Hardwareprozessoren, des erzeugten AFO-Drehmoments und der Mehrzahl von Gelenkknöchelwinkeln jeder der Mehrzahl von AFO-Steuerungen mit einem muskelskelettalen Modell der unteren Gliedmaßen (MHLLM), das ein menschliches Skelett und eine Mehrzahl von Muskeln der unteren Gliedmaßen umfasst, um ein zugehöriges integriertes AFO-MHLLM zu erzeugen, das jeder der Mehrzahl von AFO-Steuerungen entspricht;

Durchführen (312), durch den einen oder die mehreren Hardwareprozessoren, eines inversen Dynamikmechanismus an dem zugehörigen integrierten AFO-MHLLM, um ein zusätzliches Gelenkdrehmoment an der Mehrzahl von Knöchelgelenkwinkeln des menschlichen Skeletts zu berechnen, das jeder der Mehrzahl von AFO-Steuerungen entspricht;

Berechnen (314), durch den einen oder die mehreren Hardwareprozessoren, einer Mehrzahl von Muskelkräften, die der Mehrzahl von Muskeln der unteren Gliedmaßen des zugehörigen integrierten AFO-MHLLM entsprechen, für einen Gangzyklus der CP-Person unter Verwendung eines statischen Optimierungsrahmens, das jeder der Mehrzahl von AFO-Steuerungen entspricht;

Berechnen (316), durch den einen oder die mehreren Hardwareprozessoren, einer Mehrzahl von Muskelreaktionsmetriken, die einen Muskelimpuls, einen Muskelsprung, eine Muskelkoaktivierung und energetische Gehkosten umfassen, aus der Mehrzahl von Muskelkräften und dem zusätzlichen Gelenkdrehmoment, das jeder der Mehrzahl von AFO-Steuerungen entspricht;

Kombinieren (318), durch den einen oder die mehreren Hardwareprozessoren, der Mehrzahl von Muskelreaktionsmetriken, um eine AFO-Auswahlpunktzahl zu erzeugen, die jeder der Mehrzahl von AFO-Steuerungen entspricht;

Einstufen (320), durch den einen oder die mehreren Hardwareprozessoren, der Mehrzahl von AFO-Steuerungen in zunehmender Reihenfolge basierend auf den AFO-Auswahlpunktzahlen; und

Auswählen (322), durch den einen oder die mehreren Hardwareprozessoren, einer am höchsten eingestuften AFO-Steuerung als eine personalisierte optimale AFO-Steuerung aus der Mehrzahl von AFO-Steuerungen für die CP-Person.

2. Prozessorimplementiertes Verfahren nach Anspruch 1, wobei das mechanische Verhalten jeder der Mehrzahl von AFO-Steuerungen als Kombination aus einem optimalen Steifigkeitswert und einem AFO-Gleichgewichtswinkel zum Erzeugen des entsprechenden AFO-Drehmoments zusammengesetzt ist.

3. Prozessorimplementiertes Verfahren nach Anspruch 1, wobei das zusätzliche Gelenkdrehmoment basierend auf dem AFO-Gleichgewichtswinkel, dem optimalen Steifigkeitswert und der Mehrzahl von Gelenkknöchelwinkeln der CP-Person berechnet wird.

4. Prozessorimplementiertes Verfahren nach Anspruch 1, wobei das MHLLM basierend auf der Schwere des Schrittgangs entworfen wird.

5. Prozessorimplementiertes Verfahren nach Anspruch 1, wobei die Schwere des Schrittgangs eines von (i) einem normalen Gang, (ii) einem leichten Schrittgang, (iii) einem mäßigen Schrittgang und (iv) einem schweren Schrittgang umfasst.

6. System (100), das Folgendes umfasst:

   einen Speicher (102), der Anweisungen speichert;
   eine oder mehrere Kommunikationsschnittstellen (106); und
   einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen zu Folgendem konfiguriert sind:

   Empfangen von bewegungserfassten Schrittgangdaten, einer Höhe, eines Körpergewichts und einer Schwere eines Schrittgangs, der sich auf eine Person mit zerebraler Lähmung (CP) bezieht;
   Berechnen einer Gelenkknöchelwinkelkinematik, die eine Mehrzahl von Gelenkknöchelwinkeln umfasst, aus den bewegungserfassten Schrittgangdaten an einer Mehrzahl von dreidimensionalen (3D) Knöchelgelenkpositionen der CP-Person unter Verwendung einer inversen Kinematik-Pipeline;
   Zuführen der Mehrzahl von Gelenkknöchelwinkeln, der Höhe und des Körpergewichts der CP-Person zu einer Mehrzahl von Steuerungen der Knöchelfußorthese (AFO), wobei jede der Mehrzahl von AFO-Steuerungen mit einem zugehörigen mechanischen Verhalten programmiert ist;
   Berechnen eines entsprechenden AFO-Drehmoments durch jede der Mehrzahl von AFO-Steuerungen gemäß dem zugehörigen mechanischen Verhalten;
   Integrieren des erzeugten AFO-Drehmoments und der Mehrzahl von Gelenkknöchelwinkeln jeder der Mehrzahl von AFO-Steuerungen mit einem muskelskelettalen Modell der unteren Gliedmaßen (MHLLM), das ein menschliches Skelett und eine Mehrzahl von Muskeln der unteren Gliedmaßen umfasst, um ein zugehöriges integriertes AFO-MHLLM zu erzeugen, das jeder der Mehrzahl von AFO-Steuerungen entspricht;
   Durchführen eines inversen Dynamikmechanismus an dem zugehörigen integrierten AFO-MHLLM, um ein zusätzliches Gelenkdrehmoment an einem Knöchelgelenk des menschlichen Skeletts zu berechnen, das jeder der Mehrzahl von AFO-Steuerungen entspricht;
   Berechnen einer Mehrzahl von Muskelkräften, die der Mehrzahl von Muskeln der unteren Gliedmaßen des zugehörigen integrierten AFO-MHLLM entsprechen, für einen Gangzyklus der CP-Person unter Verwendung eines statischen Optimierungsrahmens, das jeder der Mehrzahl von AFO-Steuerungen entspricht;
   Berechnen einer Mehrzahl von Muskelreaktionsmetriken, die einen Muskelimpuls, einen Muskelsprung, eine Muskelkoaktivierung und energetische Gehkosten umfassen, aus der Mehrzahl von Muskelkräften und dem zusätzlichen Gelenkdrehmoment, das jeder der Mehrzahl von AFO-Steuerungen entspricht;
   Kombinieren der Mehrzahl von Muskelreaktionsmetriken, um eine AFO-Auswahlpunktzahl zu erzeugen, die jeder der Mehrzahl von AFO-Steuerungen entspricht;
   Einstufen der Mehrzahl von AFO-Steuerungen in zunehmender Reihenfolge basierend auf den AFO-Auswahlpunktzahlen; und
   Auswählen einer am höchsten eingestuften AFO-Steuerung als eine personalisierte optimale AFO-Steuerung aus der Mehrzahl von AFO-Steuerungen für die CP-Person basierend auf der Einstufung.

7. System nach Anspruch 6, wobei das mechanische Verhalten jeder der Mehrzahl von AFO-Steuerungen als Kombination aus einem optimalen Steifigkeitswert und einem AFO-Gleichgewichtswinkel zum Erzeugen des entsprechenden AFO-Drehmoments zusammengesetzt ist.

8. System nach Anspruch 6, wobei das zusätzliche Gelenkdrehmoment basierend auf dem AFO-Gleichgewichtswinkel, dem optimalen Steifigkeitswert und der Mehrzahl von Gelenkknöchelwinkeln der CP-Person berechnet wird.

9. System nach Anspruch 6, wobei das MHLLM basierend auf der Schwere des Schrittgangs entworfen wird.

10. System nach Anspruch 6, wobei die Schwere des Schrittgangs eines von (i) einem normalen Gang, (ii) einem leichten Schrittgang, (iii) einem mäßigen Schrittgang und (iv) einem schweren Schrittgang umfasst.

11. Ein oder mehrere nichtflüchtige maschinenlesbare Informationsspeichermedien, die eine oder mehrere Anweisungen umfassen, die bei Ausführung durch einen oder mehrere Hardwareprozessoren Folgendes bewirken:

> Empfangen von bewegungserfassten Schrittgangdaten, einer Höhe, eines Körpergewichts und einer Schwere eines Schrittgangs, der sich auf eine Person mit zerebraler Lähmung (CP) bezieht;
> Berechnen einer Gelenkknöchelwinkelkinematik, die eine Mehrzahl von Gelenkknöchelwinkeln umfasst, aus den bewegungserfassten Schrittgangdaten an einer Mehrzahl von dreidimensionalen Knöchelgelenkpositionen der CP-Person unter Verwendung einer inversen Kinematik-Pipeline;
> Zuführen der Mehrzahl von Gelenkknöchelwinkeln, der Höhe und des Körpergewichts der CP-Person zu einer Mehrzahl von Steuerungen der Knöchelfußorthese (AFO), wobei jede der Mehrzahl von AFO-Steuerungen mit einem zugehörigen mechanischen Verhalten programmiert ist;
> Berechnen eines entsprechenden AFO-Drehmoments durch jede der Mehrzahl von AFO-Steuerungen gemäß dem zugehörigen mechanischen Verhalten;
> Integrieren des erzeugten AFO-Drehmoments und der Mehrzahl von Gelenkknöchelwinkeln jeder der Mehrzahl von AFO-Steuerungen mit einem muskelskelettalen Modell der unteren Gliedmaßen (MHLLM), das ein menschliches Skelett und eine Mehrzahl von Muskeln der unteren Gliedmaßen umfasst, um ein zugehöriges integriertes AFO-MHLLM zu erzeugen, das jeder der Mehrzahl von AFO-Steuerungen entspricht;
> Durchführen eines inversen Dynamikmechanismus an dem zugehörigen integrierten AFO-MHLLM, um ein zusätzliches Gelenkdrehmoment an der Mehrzahl von Knöchelgelenkwinkeln des menschlichen Skeletts zu berechnen, das jeder der Mehrzahl von AFO-Steuerungen entspricht;
> Berechnen einer Mehrzahl von Muskelkräften, die der Mehrzahl von Muskeln der unteren Gliedmaßen des zugehörigen integrierten AFO-MHLLM entsprechen, für einen Gangzyklus der CP-Person unter Verwendung eines statischen Optimierungsrahmens, das jeder der Mehrzahl von AFO-Steuerungen entspricht;
> Berechnen einer Mehrzahl von Muskelreaktionsmetriken, die einen Muskelimpuls, einen Muskelsprung, eine Muskelkoaktivierung und energetische Gehkosten umfassen, aus der Mehrzahl von Muskelkräften und dem zusätzlichen Gelenkdrehmoment, das jeder der Mehrzahl von AFO-Steuerungen entspricht;
> Kombinieren der Mehrzahl von Muskelreaktionsmetriken, um eine AFO-Auswahlpunktzahl zu erzeugen, die jeder der Mehrzahl von AFO-Steuerungen entspricht;
> Einstufen der Mehrzahl von AFO-Steuerungen in zunehmender Reihenfolge basierend auf den AFO-Auswahlpunktzahlen; und
> Auswählen einer am höchsten eingestuften AFO-Steuerung als eine personalisierte optimale AFO-Steuerung aus der Mehrzahl von AFO-Steuerungen für die CP-Person.

12. Ein oder mehrere nichtflüchtige maschinenlesbare Informationsspeichermedien nach Anspruch 11, wobei das mechanische Verhalten jeder der Mehrzahl von AFO-Steuerungen als Kombination aus einem optimalen Steifigkeitswert und einem AFO-Gleichgewichtswinkel zum Erzeugen des entsprechenden AFO-Drehmoments zusammengesetzt ist.

13. Ein oder mehrere nichtflüchtige maschinenlesbare Informationsspeichermedien nach Anspruch 11, wobei das zusätzliche Gelenkdrehmoment basierend auf dem AFO-Gleichgewichtswinkel, dem optimalen Steifigkeitswert und der Mehrzahl von Gelenkknöchelwinkeln der CP-Person berechnet wird.

14. Ein oder mehrere nichtflüchtige maschinenlesbare Informationsspeichermedien nach Anspruch 11, wobei das MHLLM basierend auf der Schwere des Schrittgangs entworfen wird.

15. Ein oder mehrere nichtflüchtige maschinenlesbare Informationsspeichermedien nach Anspruch 11, wobei die Schwere des Schrittgangs eines von (i) einem normalen Gang, (ii) einem leichten Schrittgang, (iii) einem mäßigen Schrittgang und (iv) einem schweren Schrittgang umfasst.

**Revendications**

1. Procédé mis en œuvre par processeur (300), le procédé comprenant :

la réception (302), par l'intermédiaire d'un ou plusieurs processeurs matériels, de données de démarche accroupie capturées par le mouvement, d'une hauteur, d'un poids corporel et d'une gravité d'une démarche accroupie concernant un sujet de paralysie cérébrale (CP) ;

le calcul (304), par l'intermédiaire des un ou plusieurs processeurs matériels, d'une cinématique d'angle de cheville d'articulation comprenant une pluralité d'angles articulaires de la cheville, à partir des données de démarche accroupie capturées par le mouvement, au niveau d'une pluralité d'emplacements d'articulation de cheville tridimensionnels (3D) du sujet CP en utilisant un pipeline cinématique inverse ;

l'alimentation (306), par l'intermédiaire des un ou plusieurs processeurs matériels, de la pluralité d'angles articulaires de la cheville, de la hauteur et du poids corporel du sujet CP, à une pluralité de dispositifs de commande d'orthèse pied-cheville (AFO), dans lequel chacun de la pluralité de dispositifs de commande d'AFO est programmé avec un comportement mécanique associé ;

le calcul (308), par les un ou plusieurs processeurs matériels, d'un couple d'AFO correspondant, par chacun de la pluralité de dispositifs de commande d'AFO conformément au comportement mécanique associé ;

l'intégration (310), par les un ou plusieurs processeurs matériels, du couple d'AFO généré, et de la pluralité d'angles articulaires de la cheville de chacun de la pluralité de dispositifs de commande d'AFO avec un modèle de membre inférieur humain musculo-squelettique (MHLLM) comprenant un squelette humain et une pluralité de muscles de membre inférieur, pour générer un MHLLM intégré à l'AFO associé, correspondant à chacun de la pluralité de dispositifs de commande d'AFO ;

l'exécution (312), par les un ou plusieurs processeurs matériels, d'un mécanisme de dynamique inverse sur le MHLLM intégré à l'AFO associé, pour calculer un couple d'articulation supplémentaire au niveau de la pluralité d'angles articulaires de la cheville du squelette humain, correspondant à chacun de la pluralité de dispositifs de commande d'AFO ;

le calcul (314), par les un ou plusieurs processeurs matériels, d'une pluralité de forces musculaires correspondant à la pluralité de muscles de membre inférieur du MHLLM intégré à l'AFO associé, pour un cycle de démarche du sujet CP, en utilisant un cadre d'optimisation statique, correspondant à chacun de la pluralité de dispositifs de commande d'AFO ;

le calcul (316), par les un ou plusieurs processeurs matériels, d'une pluralité de métriques de réponse musculaire comprenant une impulsion musculaire, un à-coup musculaire, une co-activation musculaire et un coût énergétique de marche, à partir de la pluralité de forces musculaires et du couple d'articulation supplémentaire, correspondant à chacun de la pluralité de dispositifs de commande d'AFO ;

la combinaison (318), par les un ou plusieurs processeurs matériels, de la pluralité de métriques de réponse musculaire, pour générer un score de sélecteur d'AFO, correspondant à chacun de la pluralité de dispositifs de commande d'AFO ;

le classement (320), par les un ou plusieurs processeurs matériels, de la pluralité de dispositifs de commande d'AFO dans un ordre croissant sur la base des scores de sélecteur d'AFO ; et

la sélection (322), par les un ou plusieurs processeurs matériels, d'un dispositif de commande d'AFO le mieux classé en tant que dispositif de commande d'AFO optimal personnalisé parmi la pluralité de dispositifs de commande d'AFO pour le sujet CP.

2. Procédé mis en œuvre par processeur selon la revendication 1, dans lequel le comportement mécanique de chacun de la pluralité de dispositifs de commande d'AFO est composé en tant que combinaison d'une valeur de rigidité optimale et d'un angle d'équilibre d'AFO, pour générer le couple d'AFO correspondant.

3. Procédé mis en œuvre par processeur selon la revendication 1, dans lequel le couple d'articulation supplémentaire est calculé sur la base de l'angle d'équilibre d'AFO, de la valeur de rigidité optimale et de la pluralité d'angles articulaires de la cheville du sujet CP.

4. Procédé mis en œuvre par processeur selon la revendication 1, dans lequel le MHLLM est conçu sur la base de la gravité de la démarche accroupie.

5. Procédé mis en œuvre par processeur selon la revendication 1, dans lequel la gravité de la démarche accroupie comprend l'une parmi (i) une démarche normale, (ii) une démarche accroupie légère, (iii) une démarche accroupie modérée et (iv) une démarche accroupie sévère.

6. Système (100), comprenant :

une mémoire (102) stockant des instructions ;
une ou plusieurs interfaces de communication (106) ; et

un ou plusieurs processeurs matériels (104) couplés à la mémoire (102) via les une ou plusieurs interfaces de communication (106), dans lequel les un ou plusieurs processeurs matériels (104) sont configurés par les instructions pour :

recevoir des données de démarche accroupie capturées par le mouvement, une hauteur, un poids corporel et une gravité d'une démarche accroupie concernant un sujet de paralysie cérébrale (CP) ;

calculer une cinématique d'angle de cheville d'articulation comprenant une pluralité d'angles articulaires de la cheville, à partir des données de démarche accroupie capturées par le mouvement, au niveau d'une pluralité d'emplacements d'articulation de cheville tridimensionnels (3D) du sujet CP en utilisant un pipeline cinématique inverse ;

alimenter la pluralité d'angles articulaires de la cheville, la hauteur et le poids corporel du sujet CP, à une pluralité de dispositifs de commande d'orthèse pied-cheville (AFO), dans lequel chacun de la pluralité de dispositifs de commande d'AFO est programmé avec un comportement mécanique associé ;

calculer un couple d'AFO correspondant, par chacun de la pluralité de dispositifs de commande d'AFO conformément au comportement mécanique associé ;

intégrer le couple d'AFO généré, et la pluralité d'angles articulaires de la cheville de chacun de la pluralité de dispositifs de commande d'AFO avec un modèle de membre inférieur humain musculo-squelettique (MHLLM) comprenant un squelette humain et une pluralité de muscles de membre inférieur, pour générer un MHLLM intégré à l'AFO associé, correspondant à chacun de la pluralité de dispositifs de commande d'AFO ;

exécuter un mécanisme de dynamique inverse sur le MHLLM intégré à l'AFO associé, pour calculer un couple d'articulation supplémentaire au niveau d'une articulation de cheville du squelette humain, correspondant à chacun de la pluralité de dispositifs de commande d'AFO ;

calculer une pluralité de forces musculaires correspondant à la pluralité de muscles de membre inférieur du MHLLM intégré à l'AFO associé, pour un cycle de démarche du sujet CP, en utilisant un cadre d'optimisation statique, correspondant à chacun de la pluralité de dispositifs de commande d'AFO ;

calculer une pluralité de métriques de réponse musculaire comprenant une impulsion musculaire, un à-coup musculaire, une co-activation musculaire et un coût énergétique de marche, à partir de la pluralité de forces musculaires et du couple d'articulation supplémentaire, correspondant à chacun de la pluralité de dispositifs de commande d'AFO ;

combiner la pluralité de métriques de réponse musculaire, pour générer un score de sélecteur d'AFO, correspondant à chacun de la pluralité de dispositifs de commande d'AFO ;

classer la pluralité de dispositifs de commande d'AFO dans un ordre croissant sur la base des scores de sélecteur d'AFO ; et

sélectionner un dispositif de commande d'AFO le mieux classé en tant que dispositif de commande d'AFO optimal personnalisé parmi la pluralité de dispositifs de commande d'AFO pour le sujet CP sur la base du classement.

7. Système selon la revendication 6, dans lequel le comportement mécanique de chacun de la pluralité de dispositifs de commande d'AFO est composé en tant que combinaison d'une valeur de rigidité optimale et d'un angle d'équilibre d'AFO, pour générer le couple d'AFO correspondant.

8. Système selon la revendication 6, dans lequel le couple d'articulation supplémentaire est calculé sur la base de l'angle d'équilibre d'AFO, de la valeur de rigidité optimale et de la pluralité d'angles articulaires de la cheville du sujet CP.

9. Système selon la revendication 6, dans lequel le MHLLM est conçu sur la base de la gravité de la démarche accroupie.

10. Système selon la revendication 6, dans lequel la gravité de la démarche accroupie comprend l'une parmi (i) une démarche normale, (ii) une démarche accroupie légère, (iii) une démarche accroupie modérée et (iv) une démarche accroupie sévère.

11. Un ou plusieurs supports de stockage d'informations lisibles par machine non transitoires comprenant une ou plusieurs instructions qui, lorsqu'elles sont exécutées par un ou plusieurs processeurs matériels, amènent :

la réception de données de démarche accroupie capturées par le mouvement, d'une hauteur, d'un poids corporel et d'une gravité d'une démarche accroupie concernant un sujet de paralysie cérébrale (CP) ;

le calcul d'une cinématique d'angle de cheville d'articulation comprenant une pluralité d'angles articulaires de la cheville, à partir des données de démarche accroupie capturées par le mouvement, au niveau d'une pluralité d'emplacements d'articulation de cheville tridimensionnels du sujet CP en utilisant un pipeline cinématique inverse ;

l'alimentation de la pluralité d'angles articulaires de la cheville, de la hauteur et du poids corporel du sujet CP, à une pluralité de dispositifs de commande d'orthèse pied-cheville (AFO), dans lequel chacun de la pluralité de dispositifs de commande d'AFO est programmé avec un comportement mécanique associé ;

le calcul d'un couple d'AFO correspondant, par chacun de la pluralité de dispositifs de commande d'AFO conformément au comportement mécanique associé ;

l'intégration du couple d'AFO généré, et de la pluralité d'angles articulaires de la cheville de chacun de la pluralité de dispositifs de commande d'AFO avec un modèle de membre inférieur humain musculo-squelettique (MHLLM) comprenant un squelette humain et une pluralité de muscles de membre inférieur, pour générer un MHLLM intégré à l'AFO associé, correspondant à chacun de la pluralité de dispositifs de commande d'AFO ;

l'exécution d'un mécanisme de dynamique inverse sur le MHLLM intégré à l'AFO associé, pour calculer un couple d'articulation supplémentaire au niveau de la pluralité d'angles articulaires de la cheville du squelette humain, correspondant à chacun de la pluralité de dispositifs de commande d'AFO ;

le calcul d'une pluralité de forces musculaires correspondant à la pluralité de muscles de membre inférieur du MHLLM intégré à l'AFO associé, pour un cycle de démarche du sujet CP, en utilisant un cadre d'optimisation statique, correspondant à chacun de la pluralité de dispositifs de commande d'AFO ;

le calcul d'une pluralité de métriques de réponse musculaire comprenant une impulsion musculaire, un à-coup musculaire, une co-activation musculaire et un coût énergétique de marche, à partir de la pluralité de forces musculaires et du couple d'articulation supplémentaire, correspondant à chacun de la pluralité de dispositifs de commande d'AFO ;

la combinaison de la pluralité de métriques de réponse musculaire, pour générer un score de sélecteur d'AFO, correspondant à chacun de la pluralité de dispositifs de commande d'AFO ;

le classement de la pluralité de dispositifs de commande d'AFO dans un ordre croissant sur la base des scores de sélecteur d'AFO ; et

la sélection d'un dispositif de commande d'AFO le mieux classé en tant que dispositif de commande d'AFO optimal personnalisé parmi la pluralité de dispositifs de commande d'AFO pour le sujet CP.

12. Un ou plusieurs supports de stockage d'informations lisibles par machine non transitoires selon la revendication 11, dans lequel le comportement mécanique de chacun de la pluralité de dispositifs de commande d'AFO est composé en tant que combinaison d'une valeur de rigidité optimale et d'un angle d'équilibre d'AFO, pour générer le couple d'AFO correspondant.

13. Un ou plusieurs supports de stockage d'informations lisibles par machine non transitoires selon la revendication 11, dans lequel le couple d'articulation supplémentaire est calculé sur la base de l'angle d'équilibre d'AFO, de la valeur de rigidité optimale et de la pluralité d'angles articulaires de la cheville du sujet CP.

14. Un ou plusieurs supports de stockage d'informations lisibles par machine non transitoires selon la revendication 11, dans lequel le MHLLM est conçu sur la base de la gravité de la démarche accroupie.

15. Un ou plusieurs supports de stockage d'informations lisibles par machine non transitoires selon la revendication 11, dans lequel la gravité de la démarche accroupie comprend l'une parmi (i) une démarche normale, (ii) une démarche accroupie légère, (iii) une démarche accroupie modérée et (iv) une démarche accroupie sévère.

**SYSTEM**

100

MEMORY

102

DATABASE

108

HARDWARE

PROCESSOR(S)

104

I/O INTERFACE(S)

106

**FIG. 1**

**FIG. 2**

300

receiving a motion-captured crouch gait data, a height, a body weight, and a severity of a crouch gait pertaining to a Cerebral Palsy (CP) subject **302**

↓

computing a joint ankle angle kinematics comprising a plurality of joint ankle angles, from the motion-captured crouch gait data, at three-dimensional (3D) ankle joint locations of the CP subject using an inverse kinematics pipeline **304**

↓

feeding the plurality joint ankle angles, the height, and the body weight of the CP subject, to a plurality of Ankle Foot Orthosis (AFO) controllers **306**

↓

computing a corresponding AFO torque, by each of the AFO controllers in accordance with an associated mechanical behaviour **308**

↓

integrating the generated AFO torque, and the plurality joint ankle angles of each of the plurality of AFO controllers with a musculoskeletal human lower limb model (MHLLM) comprising a human skeleton and a plurality of  lower limb muscles, to generate an associated AFO integrated MHLLM corresponding to each of the plurality of AFO controllers **310**

↓

(A)

**FIG. 3A**

300 ⟍         Ⓐ

performing an inverse dynamics mechanism on the associated AFO integrated MHLLM, to compute an additional joint torque at the plurality of joint ankle angles of the human skeleton, corresponding to each of the plurality of AFO controllers **312**

↓

computing a plurality of muscle forces corresponding to the lower limb muscles of AFO integrated MHLLM, for a gait cycle of the CP subject, using a static optimization framework, corresponding to each of the plurality of AFO controllers **314**

↓

computing a plurality of muscle response metrics comprising a muscle impulse, a yank, a muscle coactivation, and an energetic cost of walking, from the plurality of muscle forces and the additional joint torque, corresponding to each of the plurality of AFO controllers **316**

↓

combining the plurality of muscle response metrics, to generate an AFO selector score, corresponding to each of the plurality of AFO controllers **318**

↓

ranking the plurality of AFO controllers based on the AFO selector scores **320**

↓

Selecting a top ranked AFO controller as a personalized optimal AFO controller from among the plurality of AFO controllers for the CP subject based on the ranking **322**

**FIG. 3B**

**FIG. 4A**

**FIG. 4B**

**Muscle Force (Severe crouch)**

FIG. 4C

FIG. 5

**FIG. 6**

**FIG. 7**

**FIG. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421021181

**Non-patent literature cited in the description**

- **STEELE KATHERINE M et al.** How much muscle strength is required to walk in a crouch gait?. *JOURNAL OF BIOMECHANICS*, vol. 45 (15), 2564-2569
- A Framework of a Lower Limb Musculoskeletal Model With Implemented Natural Proprioceptive Feedback and Its Progressive Evaluation. **ZHANG HAOTIAN et al.** IEEE TRANSACTIONS ON NEURAL SYSTEMS AND REHABILITATION ENGINEERING. IEEE, 19 June 2020, vol. 28, 1866-1875
- Effect of Ankle-Foot Orthosis Stiffness on Muscle Force During Gait Through Mechanical Testing and Gait Simulation. **YAMAMOTO MASATAKA et al.** IEEE ACCESS. IEEE, 08 July 2021, vol. 9, 98039-98047